# DEMANDE DE BREVET EUROPEEN

(11) **EP 1 502 579 A1**
(43) Date de publication de la demande: **02.02.2005**
(21) Numéro de dépôt: 04103638.5
(22) Date de dépôt: 28.07.2004
(51) Int. Cl.: A61K 7/13

(54) **Composition de teinture de fibres keratiniques comprenant un colorant direct azodiazine particulier et procedes de coloration la mettant en oeuvre**

(30) Priorité: 30.07.2003 FR 0350387
(71) Demandeur: L'OREAL, 75008 Paris (FR)
(72) Inventeur: Lagrange, Alain, 77700 Coupvray (FR); Guerin, Frédéric, 75009 Paris (FR); Kravtchenko, Sylvain, 92600 Asnieres (FR)
(74) Mandataire: Poulin, Gérard

(57) **Abrégé**

La présente invention a trait à une composition de teinture des fibres kératiniques comprenant au moins un colorant direct particulier répondant à la formule (I) suivante : 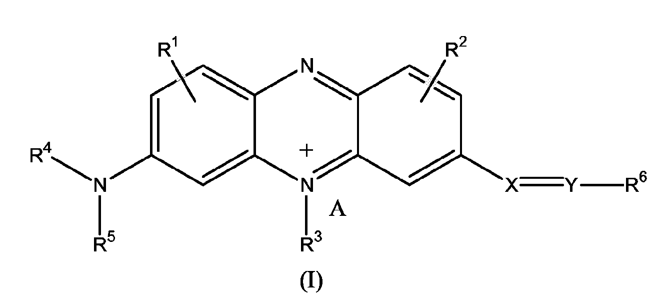 dans laquelle R¹ et R² peuvent représenter un atome hydrogène, R³ peut représenter un groupe phényle, R⁴ et R⁵ peuvent représenter un groupe alkyle et R⁶ peut représenter un groupe cyclique cationique comprenant, par exemple, un un cycle N-alkyl(C1-C4)pyridinium, un cycle N-alkyl(C1-C4)imidazolium, un cycle N-alkyl(C1-C4)triazolium, X et Y peuvent représenter un atome d'azote, A est un contre-ion anionique.

La présente invention a également trait à un procédé de coloration des fibres kératiniques mettant en oeuvre ces colorants directs particuliers.

## Description

### DOMAINE TECHNIQUE

La présente invention se rapporte à des compositions de teinture des fibres kératiniques, en particulier de fibres kératiniques humaines et plus spécialement de cheveux, comprenant au moins un colorant direct particulier appartenant à la famille des composés azodiazines.

Elle se rapporte également à l'utilisation de composés particuliers de la famille des azodiazines en tant que colorants directs dans des compositions de teinture de fibres kératiniques.

Enfin, la présente invention a trait à des procédés de coloration de fibres kératiniques mettant en oeuvre de telles compositions.

### ETAT DE LA TECHNIQUE ANTERIEURE

Pour colorer les fibres kératiniques, tels que les cheveux, il est connu d'utiliser des compositions tinctoriales contenant des précurseurs de colorant d'oxydation (en particulier des ortho ou paraphénylènediamines, des ortho- ou paraaminophénols, généralement appelés « bases d'oxydation »)et éventuellement des coupleurs (métaphénylènediamines, métaaminophénols et métadiphénols encore appelés modificateurs de coloration). Les précurseurs de colorants d'oxydation sont des précurseurs incolores ou faiblement colorés qui, associés à des produits oxydants (telle que l'eau oxygénée) donnent naissance par un processus d'oxydation à des composés colorés et colorants.

Toutefois, les procédés de coloration par oxydation présentent les inconvénients suivants :
- du fait de l'utilisation de produits oxydants tels que l'eau oxygénée, ils peuvent engendrer une dégradation de la fibre kératinique ainsi qu'une irritation gênante du cuir chevelu ;
- ils engendrent une coloration tenace des fibres, laquelle peut virer avec le temps et ils entraînent souvent une sélectivité de coloration de la fibre, c'est-à-dire des écarts de coloration le long d'une même fibre kératinique.

Pour s'affranchir des inconvénients mentionnés ci-dessus, il a été proposé de recourir à des procédés de coloration directe par l'emploi de colorants directs, qui consiste à colorer les cheveux en faisant pénétrer une molécule colorée (le colorant direct) par diffusion à l'intérieur du cheveu sans recourir à l'emploi d'eau oxygénée.

Toutefois, de tels procédés se sont révélés, jusqu'à présent, insatisfaisants notamment pour les raisons suivantes :
- ils engendrent une ténacité de coloration insuffisante, laquelle coloration se dissipe au bout de quelques shampooings ;
- ils engendrent eux aussi une sélectivité de la coloration des fibres, c'est-à-dire des écarts de coloration le long d'une même fibre kératinique.

Il existe donc un véritable besoin pour une composition de teinture des fibres kératiniques, qui soit peu sélective, qui puisse donner une grande variété de couleurs, des couleurs puissantes et qui permette, en outre, de donner une coloration des fibres tenace, qui vire peu au cours du temps.

Ainsi, la Demanderesse a découvert, de manière surprenante, que certains composés azodiazines incorporés dans des compositions de teinture des fibres kératiniques permettent de surmonter les inconvénients rencontrés dans l'art antérieur et permettent notamment d'obtenir une gamme de couleurs très variées et une sélectivité très faible ainsi qu'un bon niveau de ténacité.

De façon plus précise, l'invention a pour objet une composition de teinture des fibres kératiniques comprenant au moins un colorant choisi parmi les composés de formule (I) suivante: 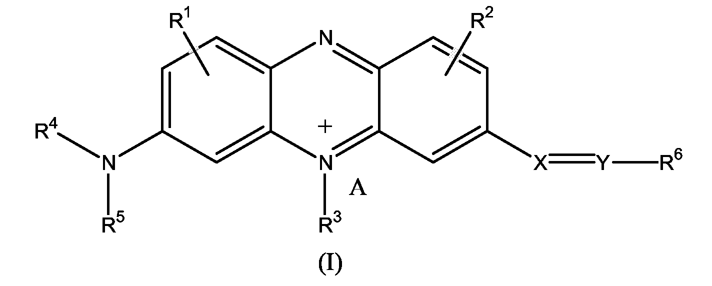 dans laquelle :
- R¹ et R² représentent indépendamment l'un de l'autre :
   - un atome d'hydrogène ;
   - un groupe alkyle comportant de 1 à 6 atomes de carbone, ledit groupe pouvant être éventuellement substitué par un ou plusieurs groupes choisis parmi les groupes hydroxyle, amino, halogène, aryle, alcoxy en C1 à C3 ;
   - un groupe aryle comportant de 6 à 18 atomes de carbone, ledit groupe aryle pouvant être éventuellement substitué par un ou plusieurs groupes choisis parmi les groupes amino, hydroxy, alcoxy en C1 à C3, un groupe alkyle en C1 à C6 ;
   - un groupe carboxyalkyle comportant de 1 à 6 atomes de carbone ; ou
   - un groupe sulfoalkyle comportant de 1 à 6 atomes de carbone ;
- R³ représente un atome d'hydrogène, un groupe alkyle comportant de 1 à 6 atomes de carbone éventuellement substitué, un groupe alcényle éventuellement substitué, ou un groupe aryle éventuellement susbtitué par un ou plusieurs groupes choisis parmi les groupes hydroxyle, amino, halogène, alcoxy ou alkyle en C1 à C3 ;
- R⁴ et R⁵ représentent indépendamment l'un de l'autre un atome d'hydrogène, un groupe aryle ou un groupe alkyle comportant de 1 à 6 atomes de carbone éventuellement substitué par un ou plusieurs groupes choisis parmi les groupes hydroxyles, cyano, halogène, amino, aryle ou alcoxy comportant de 1 à 4 atomes de carbone ;
- R⁶ représente un groupe mono- ou polycyclique comportant de 5 à 100 atomes de carbone et éventuellement un ou plusieurs hétéroatomes et éventuellement une ou plusieurs insaturations, ledit groupe mono- ou polycyclique pouvant être substitué par un ou plusieurs groupes choisis parmi les groupes hydroxy, halogène, amino, alcoxy, alkyle, aromatique, à condition que le groupe R⁶ ne soit pas un groupe phénol, aniline, diphénylamine, acylacétoarylamide, pyridone, quinoline, isoquinoline, pyrazole, indole, aminopyridine, pyrimidine, pyrimidone, naphtol, naphtylamine, aminothiazole, thiophène, hydroxypyridine ;
- X et Y représentent, indépendamment l'un de l'autre un atome d'azote ou un groupe -CR' avec R' représentant un atome d'hydrogène ou un groupe alkyle comportant de 1 à 6 atomes de carbone;
- A est un contre-ion anionique.

Selon l'invention, on précise que, dans ce qui précède et ce qui suit, l'on entend généralement par groupe alkyle un groupe alkyle linéaire ou ramifié de 1 à 6 atomes de carbone, par exemple un groupe méthyle, éthyle, n-propyle, i-propyle, n-butyle, i-butyle, t-butyle.

Par groupe alcoxy, on entend généralement, dans ce qui précède et ce qui suit, un groupe -O- alkyle, le terme alkyle répondant à la même définition que celle donnée ci-dessus.

Par groupe alcényle, on entend généralement, dans ce qui précède et ce qui suit, un groupe alcényle linéaire ou ramifié de 2 à 20 atomes de carbone.

Des exemples de groupes alcényle sont les groupes vinyle, allyle, cyclohexényle.

Par groupe aryle, on entend généralement, dans ce qui précède et ce qui suit, un groupe aromatique hydrocarboné monocyclique ou polycyclique comprenant de 6 à 18 atomes de carbone, tel que le groupe phényle, le groupe naphtyle. Ce groupe peut être éventuellement substitué par des groupes choisis parmi les groupes amino, hydroxy, alcoxy en C1 à C3, un groupe alkyle en C1 à C6. Des exemples de groupes aryle substitués sont, par exemple, le groupe 2-tolyle, 3-tolyle, 4-tolyle.

Par groupe carboxyalkyle, on entend généralement, dans ce qui précède et ce qui suit, un groupe alkyle tel que défini précédemment comportant en extrémité un groupe -CO₂H, tel que le groupe carboxyméthyle -CH₂-CO₂H, le groupe carboxyéthyle -(CH₂)₂-CO₂H.

Par groupe sulfoalkyle, on entend généralement, dans ce qui précède et ce qui suit, un groupe alkyle tel que défini précédemment comportant un atome de soufre formant pont entre le groupe alkyle susmentionné et le motif tricyclique des composés de formule (I).

Par groupe amino, on entend généralement, dans ce qui précède et ce qui suit, un groupe de formule -NH₂, éventuellement substitué au niveau de l'atome d'azote par un ou deux substituants tels que des groupes alkyles comportant de 1 à 6 atomes de carbone.

Par contre ion anionique, on entend, selon l'invention, un anion apte à neutraliser la charge positive portée par l'atome d'azote chargé positivement du motif tricyclique des composés de formule (I). Ce contre-ion peut être un halogénure (tel qu'un chlorure, bromure, iodure), un sulfate, un méthosulfate, un phosphate, un tosylate.

Par hétéroatome, on entend, dans ce qui précède et ce qui suit, un atome autre qu'un atome de carbone, tel qu'un atome d'oxygène, d'azote, de soufre.

Selon l'invention, R³ peut représenter un groupe alkyle éventuellement substitué, par exemple, par un groupe hydroxyle, un groupe cyano, un groupe halogène, un groupe amino, un groupe aryle, un groupe alcoxy comprenant de 1 à 4 atomes de carbone. Il peut représenter également un groupe alcényle tel que défini précédemment, éventuellement substitué, par exemple, par un groupe hydroxyle, un groupe cyano, un groupe halogène, un groupe amino, un groupe aryle, un groupe alcoxy comprenant de 1 à 4 atomes de carbone, un groupe alkyle. Enfin, R³ peut représenter un groupe aryle comportant généralement de 6 à 18 atomes de carbone, éventuellement substitué par un ou plusieurs substituants choisis parmi les groupes hydroxyle, halogène, amino, alcoxy ou alkyle en C1 à C3. A titre d'exemple, R³ peut représenter un groupe phényle éventuellement substitué.

De préférence, le groupe R⁶ tel que défini précédemment est un groupe cationique. Avantageusement, un tel groupe est un groupe N-alkyl(C1-C4)pyridinium, un groupe N-alkyl(C1-C4)imidazolium, un groupe N-alkyl(C1-C4)triazolium, lesdits groupes pouvant être éventuellement substitués.

Les composés de formule (I) peuvent être définis comme des colorants directs, c'est-à-dire ne nécessitent pas de révélation par un agent approprié telle que les colorants d'oxydation.

Parmi les composés de formule (I) définie ci-dessus, on préfère utiliser, avantageusement, les composés de formules (II) et (III) suivantes, dans lesquels A désigne un contre-ion anionique, de préférence, un anion chlorure et W désigne un contre-ion anionique, de préférence un anion chlorure :

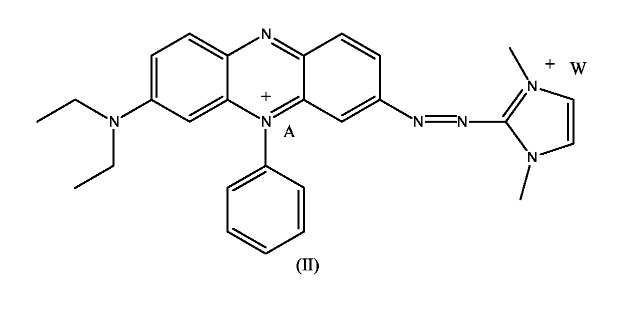

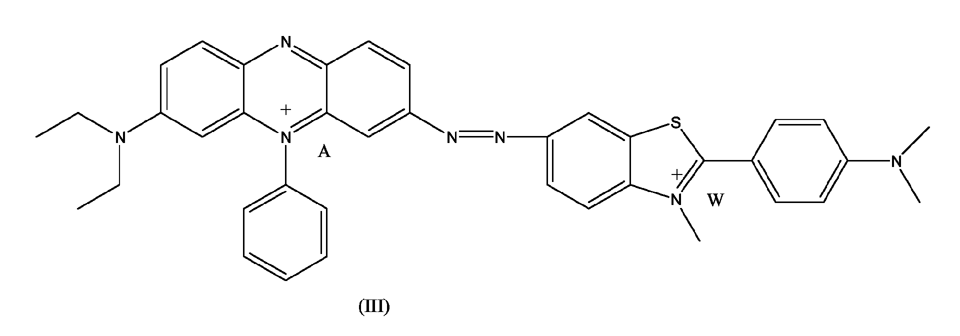

Ces colorants peuvent être obtenus par des schémas classiques de synthèse comme ceux décrits dans le brevet FR 1285848.

Il s'avère que les colorants de l'invention permettent d'obtenir des colorations intenses sur cheveux naturels ou éventuellement sensibilisés.

Ces colorants permettent également d'obtenir des reflets variés chromatiques ou sombres, très puissants, peu sélectifs et présentant un bon niveau de ténacité.

En particulier, les colorants de l'invention permettent d'obtenir des reflets gris neutres et noirs, qui virent peu au cours du temps.

La teneur en colorant(s) de formule (I) peut varier entre 0,001 et 20% en poids environ par rapport au poids total de la composition de teinture et de préférence entre environ 0,01 et 10% et encore plus préférentiellement entre environ 0,1 et 5%.

La composition de teinture selon l'invention est de préférence composée d'un milieu aqueux constitué par de l'eau ou par un mélange d'eau et d'un solvant organique acceptable sur le plan cosmétique. On peut citer à titre d'exemples de solvant organiques acceptables sur le plan cosmétique des alcools tels que l'alcool éthylique, l'alcool isopropylique, l'alcool benzylique, des polyols, des éthers de polyols, des alcanes, des cétones et des mélanges de ceux-ci.

En outre, la composition peut comprendre un ou plusieurs colorants directs différents des composés de formule (I) définis ci-dessus. Ces colorants directs peuvent être choisis parmi les colorants directs classiquement utilisés en coloration directe. On peut citer parmi ces colorants les colorants aromatiques et/ou non aromatiques d'utilisation courante tels que les colorants nitrés, les méthines, les azométhines, les styriles, les triarylméthanes, les diarylméthanes, les colorants azoïques, les colorants anthraquinoniques et naphtoquinoniques, les porphyrines, les tétraphénylporphyrines, les métalloporphyrines, les phtalocyanines, les colorants naturels de type caroténoïdes, terpénoïdes, flavonoïdes, les colorants fluorescents tels que la fluorescéine, la rhodamine, la coumarine.

La composition de la présente invention peut comprendre, en outre, une ou plusieurs bases d'oxydation associées éventuellement avec un ou plusieurs coupleurs utilisés classiquement pour la coloration par oxydation.

A titre d'exemples de bases d'oxydation, on peut citer les paraphénylènediamines, les bisphénylalkylènediamines, les paraaminophénols, les orthoaminophénols, les bases hétérocycliques.

Les coupleurs associés peuvent être des coupleurs métaphénylènediamines, les coupleurs métaaminophénols, les coupleurs métadiphénols, les coupleurs naphtaléniques, les coupleurs hétérocycliques.

Outre les colorants, la composition de teinture de l'invention peut comprendre également les additifs usuels des compositions de teinture, ces additifs pouvant être choisis parmi les agents tensioactifs, les agents épaississants, les agents anti-oxydants, les agents séquestrants, les agents dispersants, les agents de conditionnement du cheveu, les agents conservateurs, les agents opacifiants, les agents acidifiants, les agents alcalinisants, les parfums.

Il est entendu que l'homme du métier effectuera un choix approprié de ces additifs, de manière à ce que les propriétés avantageuses de la composition inhérentes à la présence des composés de formule (I) tels que définis précédemment ne soient pas altérés par les additifs mentionnés ci-dessus.

Le ou les agents tensioactifs susceptibles d'être présents dans la composition peuvent être indifféremment choisis parmi les tensioactifs anioniques, non ioniques, amphotères et cationiques.

Des agents tensioactifs anioniques, non ioniques, amphotères ou cationiques convenant à la mise en oeuvre de l'invention sont notamment les suivants :

### • tensioactifs anioniques :

A titre d'exemples d'agents tensioactifs anioniques susceptibles d'être utilisés, seuls ou en mélanges, on peut citer les sels, en particulier les sels alcalins (sels de sodium, sels de magnésium, sels d'ammonium, sels d'amines, sels d'aminoalcools, ...) des composés suivants : alkylsulfates, alkyléthersulfates, alkylamidoéthersulfates, alkylarylpolyéthersulfates, monoglycérides sulfates ; alkylsulfonates, alkylphosphates, alkylamidesulfonates, alkylarylsulfonates, α-oléfine-sulfonates, paraffine-sulfonates ; alkyl(C₆-C₂₄)sulfosuccinates, alkyl (C₆-C₂₄)éthersulfosuccinates, alkyl(C₆-C₂₄)amidesulfosuccinates, alkyl(C₆-C₂₄)sulfoacétates ; acyl (C₆-C₂₄)sarcosinates et acyl (C₆-C₂₄)-glutamates.

On peut aussi citer les esters d'alkyl(C₆-C₂₄)polyglycosides carboxyliques tels que les alkylpolyglucoside citrates, les alkylpolyglucoside tartrates, les alkylpolyglucoside sulfosuccinates et les alkylpolyglucoside sulfosuccinamates ; les acyliséthionates et les N-acyltaurates, le radical alkyle ou acyle de tous ces composés comportant, de préférence, de 12 à 20 atomes de carbone, et le radical aryle désignant, de préférence, un groupement phényle ou benzyle.

Sont également utilisables les sels d'acides gras tels que les sels des acides oléique, ricinoléique, palmitique et stéarique, des acides d'huile de coprah ou d'huile de coprah hydrogénée ; les acyllactylates dont le radical acyle comporte de 8 à 20 atomes de carbone ; les acides d'alkyl D galactoside uroniques et leurs sels ; les acides alkyl(C₆-C₂₄)éther carboxyliques polyoxyalkylénés, les acides alkyl(C₆-C₂₄)amido éther carboxyliques polyoxyalkylénés et leurs sels, en particulier ceux comportant de 2 à 50 groupes oxydes d'alkylène et, plus spécialement oxydes d'éthylène, et leurs mélanges.

### • tensioactifs non ioniques :

Les agents tensioactifs non ioniques sont des composés bien connus en eux-mêmes (voir, par exemple, le "Handbook of Surfactants", M.R. PORTER, Ed. Blackie & Son, Glasgow and London, 1991, 116-178) et leur nature ne revêt pas, dans le cadre de la présente invention, de caractère critique.

Ainsi, utilisés seuls ou en mélanges, ils peuvent notamment être choisis parmi les alcools, les α-diols, les alkylphénols polyéthoxylés et polypropoxylés ayant une chaîne grasse comportant, par exemple, de 8 à 18 atomes de carbone, le nombre de groupes oxydes d'éthylène ou oxydes de propylène pouvant être notamment de 2 à 50 ; les copolymères d'oxyde d'éthylène et de propylène, les condensats d'oxyde d'éthylène et de propylène sur des alcools gras, les amides gras polyéthoxylés ayant, de préférence, de 2 à 30 moles d'oxyde d'éthylène ; les amides gras polyglycérolés comportant en moyenne de 1 à 5 et, plus spécialement, de 1,5 à 4, groupes glycérol ; les esters d'acide gras du sorbitan oxyéthylénés comportant de 2 à 30 moles d'oxyde d'éthylène ; les esters d'acides gras du sucrose, les esters d'acides gras du polyéthylèneglycol ; les alkylpolyglycosides ; les dérivés de N-alkyl glucamine et les oxydes d'amines tels que les oxydes d'alkyl(C₁₀-C₁₄) amines ou les oxydes de N-acylaminopropylmorpholine.

### • tensioactifs amphotères :

Les agents tensioactifs amphotères (ou zwittérioniques), dont la nature ne revêt pas de caractère critique dans le cadre de la présente invention, peuvent notamment être choisis, seuls ou en mélanges, parmi les dérivés d'amines secondaires ou tertiaires aliphatiques dont le radical aliphatique est une chaîne linéaire ou ramifiée comportant de 8 à 18 atomes de carbone et contenant au moins un groupe anionique hydrosolubilisant, par exemple un carboxylate, un sulfonate, un sulfate, un phosphate ou un phosphonate.

On peut également citer les alkyl(C₈-C₂₀)bétaïnes, les sulfobétaïnes, les alkyl(C₈-C₂₀)amidoalkyl(C₁-C₆)bétaïnes et les alkyl(C₈-C₂₀)amidoalkyl(C₁-C₆)sulfobétaïnes.

Parmi les dérivés d'amines, on peut notamment citer les composés commercialisés par la société Rhodia Chimie sous la dénomination commerciale Miranol®, qui sont décrits dans US 2,528,378 et US 2,781,354 et qui sont classés dans le Dictionnaire CTFA, 5^{ème} édition, 1993, sous les désignations anglo-saxonnes "disodium cocoamphodiacetate", "disodium lauroamphodiacetate", "disodium caprylamphodiacetate", "disodium capryloamphodiacetate", "disodium cocoamphodipropionate", "disodium lauroamphodipropionate", "disodium caprylamphodipropionate", "disodium capryloamphodipropionate", "lauroamphodiproponic acid" et "cocoamphodipropionic acid".

### • tensioactifs cationiques :

Comme agents tensioactifs cationiques aptes à être utilisés seuls ou en mélanges, on peut citer les sels d'amines grasses primaires, secondaires et tertiaires, éventuellement polyoxyalkylénées ; les sels d'ammonium quaternaire tels que les chlorures et les bromures de tétraalkylammonium, d'alkylamidoalkyltrialkylammonium, de trialkylbenzylammonium, de trialkylhydroxyalkylammonium et d'alkylpyridinium ; les dérivés d'imidazoline et les oxydes d'amines à caractère cationique.

Les agents épaississants pouvant être incorporés dans les compositions de l'invention peuvent être d'origine minérale ou organique. Parmi ceux-ci, on peut citer les polymères épaississants d'origine naturelle telles que les gommes (gomme de xanthane, gomme de caroube, gomme de guar), les polymères épaississants d'origine synthétique (tels que l'hydroxyéthylcellulose, les polyacides acryliques). Parmi ces polymères synthétiques, on citera tout particulièrement les polymères associatifs comprenant une chaîne grasse tel que les polymères associatifs de type acrylique ou polyuréthane.

Le pH de la composition de teinture conforme à l'invention est généralement compris entre 3 et 12 environ, de préférence entre 5 et 11 et encore plus préférentiellement entre 6 et 10.

Ce pH peut être ajusté à la valeur souhaitée grâce à l'ajout à la composition d'agents acidifiants ou alcalinisants utilisés généralement en teinture des fibres kératiniques ou bien encore à l'aide de systèmes tampons classiques.

Parmi les agents acidifiants, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants, on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule (IV) suivante : 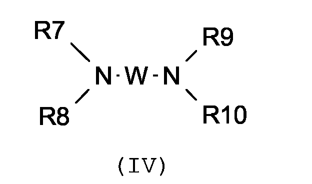 dans laquelle W est un reste propylène éventuellement substitué par un groupement hydroxyle ou un radical alkyle en C₁-C₄ ; R₇, R₈, R₉ et R₁₀, identiques ou différents, représentent un atome d'hydrogène, un radical alkyle en C₁-C₄ ou hydroxyalkyle en C₁-C₄.

La composition cosmétique peut se présenter sous des formes galéniques diverses, telles qu'une lotion, une crème, un gel ou tout autre forme appropriée pour réaliser une teinture des fibres kératiniques. Elle peut également être conditionnée sous pression en flacon aérosol en présence d'un agent propulseur et former une mousse.

L'invention a également trait à l'utilisation de composés de formule (I) telle que définie ci-dessus en tant que colorant direct dans des compositions de teinture des fibres kératiniques, en particulier des fibres kératiniques humaines, telles que les cheveux.

On précise que la composition répond aux mêmes spécifications que la composition décrite ci-dessus.

Enfin, l'invention a enfin trait à un procédé de coloration directe de fibres kératiniques, comprenant successivement les étapes suivantes :
a) appliquer sur les fibres kératiniques une composition de teinture telle que définie précédemment;
b) laisser reposer la composition sur les fibres kératiniques pendant un temps de repos suffisant pour obtenir la coloration;
c) rincer éventuellement les fibres kératiniques pour en éliminer ladite composition de teinture;
d) laver éventuellement les fibres kératiniques une ou plusieurs fois, les rincer après chaque lavage
e) les sécher.

Ainsi, le procédé de coloration directe de l'invention comprend une première étape consistant à appliquer sur les cheveux à teindre la composition de teinture telle que définie précédemment puis, selon une deuxième étape à la laisser reposer, généralement pendant un temps de repos de 3 à 60 minutes, de préférence de 5 à 40 minutes, et plus préférentiellement encore de 15 à 30 minutes, de façon à bien laisser le temps à la composition d'agir correctement sur les cheveux. Cette phase de repos peut être effectuée à une température allant de la température ambiante à 80 °C, de préférence de 25 à 55°C.

Ensuite, les fibres kératiniques ainsi colorés sont éventuellement rincées pour éliminer la composition de teinture ayant réagi avec les fibres et éventuellement lavées une ou plusieurs fois.

Lorsque la composition de teinture comporte au moins un composé de formule (I) et au moins un colorant d'oxydation, tel que mentionné précédemment, le procédé de teinture nécessite une étape supplémentaire de révélation par un agent oxydant de la couleur du colorant d'oxydation.

Par conséquent, l'invention a également trait à un procédé de coloration des fibres kératiniques comprenant successivement les étapes suivantes :
f) appliquer sur les fibres kératiniques une composition de teinture comprenant au moins un composé de formule (I) telle que définie précédemment et au moins un colorant d'oxydation, la couleur du colorant d'oxydation étant révélée par un agent oxydant ;
g) laisser reposer la composition sur les fibres kératiniques pendant un temps de repos suffisant pour obtenir la coloration;
h) rincer éventuellement les fibres kératiniques pour en éliminer ladite composition de teinture;
i) laver éventuellement les fibres kératiniques une ou plusieurs fois, les rincer après chaque lavage ;
j) les sécher.

Les agents oxydants utilisables sont, par exemple, le peroxyde d'hydrogène, le peroxyde d'urée, les bromates de métaux alcalins, les persels tels que les perborates et persulfates, les peracides et les enzymes oxydases telles que les peroxydases, les oxydoréductases à deux électrons telles que les uricases et les oxygénases à quatre électrons telles que les laccases. De préférence, l'agent oxydant est le peroxyde d'hydrogène.

On précise que l'agent oxydant peut être ajouté à la composition de l'invention juste au moment de l'emploi ou il peut être mis en oeuvre à partir d'une composition oxydante le contenant.

On précise enfin que la composition est laissée à reposer, généralement de 3 à 60 minutes, de préférence de 5 à 40 minutes, et plus préférentiellement encore de 15 à 30 minutes, de façon à bien laisser le temps à la composition d'agir correctement sur les cheveux et à la révélation d'avoir lieu. Cette phase de repos peut être effectuée à une température allant de la température ambiante à 80 °C, de préférence de 25 à 55°C.

L'invention va maintenant être décrite au regard des exemples suivants, donnés à titre illustratif et non limitatif.

### EXEMPLE 1.

Une composition tinctoriale 1 conforme à l'invention présente les constituants explicités dans le tableau 1 ci-dessous. Cette composition comprend un colorant (1) (Poids molaire : 488 g/mol) conforme à l'invention répondant à l'une des formules suivantes :

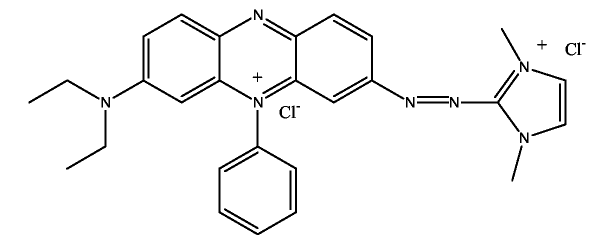

**TABLEAU 1**

| Constituants | Quantité |
|---|---|
| Colorant (1) | 0,49 g |
| Alcool benzylique | 4 g |
| Polyéthylèneglycol 6OE | 6 g |
| Hydroxyléthylcellulose | 0,7 g |
| Alkylpolyglucoside en solution aqueuse à 60% MA* | 4,5 g MA* |
| Tampon phosphate | qs pH 7 |
| Eau déminéralisée | qsp 100 g |

| | |
|---|---|
| *MA : Matière active. | |

### EXEMPLE 2.

Une composition tinctoriale 2 conforme à l'invention présente les constituants explicités dans le tableau 2 ci-dessous. Cette composition comprend un colorant (2) (Poids molaire : 660 g/mol) conforme à l'invention répondant à l'une des formules suivantes :

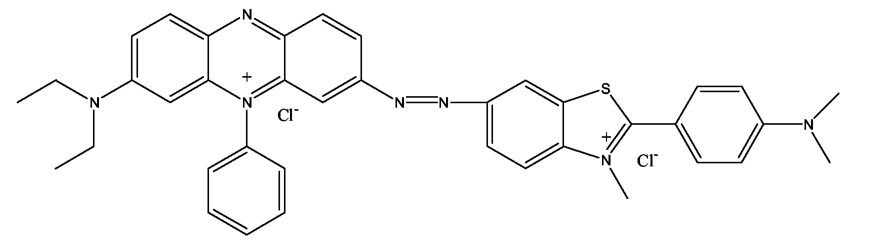

**TABLEAU 2**

| Constituants | Quantité |
|---|---|
| Colorant (2) | 0,66 g |
| Alcool benzylique | 4 g |
| Polyéthylèneglycol 6OE | 6 g |
| Hydroxyléthylcellulose | 0,7 g |
| Alkylpolyglucoside en solution aqueuse à 60% MA* | 4,5 g MA* |
| Tampon phosphate | qs pH 7 |
| Eau déminéralisée | qsp 100 g |

| | |
|---|---|
| *MA : Matière active. | |

## Revendications

1. Composition de teinture des fibres kératiniques comprenant au moins un colorant choisi parmi les composés de formule (I) suivante: 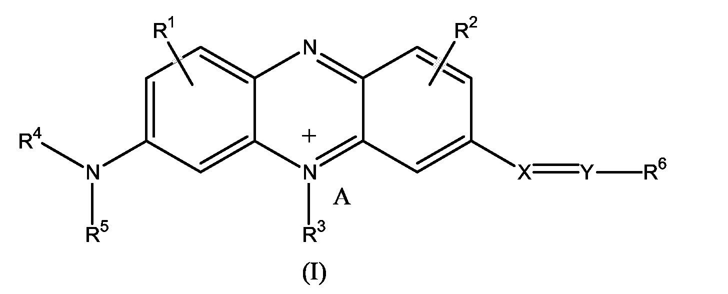 dans laquelle :
- R¹ et R² représentent indépendamment l'un de l'autre :
- un atome d'hydrogène ;
- un groupe alkyle comportant de 1 à 6 atomes de carbone, ledit groupe pouvant être éventuellement substitué par un ou plusieurs groupes choisis parmi les groupes hydroxyle, amino, halogène, aryle, alcoxy en C1 à C3 ;
- un groupe aryle comportant de 6 à 18 atomes de carbone, ledit groupe aryle pouvant être éventuellement substitué par un ou plusieurs groupes choisis parmi les groupes amino, hydroxy, alcoxy en C1 à C3, un groupe alkyle en C1 à C6 ;
- un groupe carboxyalkyle comportant de 1 à 6 atomes de carbone ; ou
- un groupe sulfoalkyle comportant de 1 à 6 atomes de carbone ;
- R³ représente un atome d'hydrogène, un groupe alkyle comportant de 1 à 6 atomes de carbone éventuellement substitué, un groupe alcényle éventuellement substitué, ou un groupe aryle éventuellement susbtitué par un ou plusieurs groupes choisis parmi les groupes hydroxyle, amino, halogène, alcoxy ou alkyle en C1 à C3;
- R⁴ et R⁵ représentent indépendamment l'un de l'autre un atome d'hydrogène, un groupe aryle ou un groupe alkyle comportant de 1 à 6 atomes de carbone, éventuellement substitué par un ou plusieurs groupes choisis parmi les groupes hydroxy, cyano, halogène, amino, aryle ou alcoxy comportant de 1 à 4 atomes de carbone ;
- R⁶ représente un groupe mono- ou polycyclique comportant de 5 à 100 atomes de carbone et éventuellement un ou plusieurs hétéroatomes et éventuellement une ou plusieurs insaturations, ledit groupe mono- ou polycyclique pouvant être substitué par un ou plusieurs groupes choisis parmi les groupes hydroxy, halogène, amino, alcoxy, alkyle, aromatique, à condition que le groupe R⁶ ne soit pas un groupe phénol, aniline, diphénylamine, acylacétoarylamide, pyridone, quinoline, isoquinoline, pyrazole, indole, aminopyridine, pyrimidine, pyrimidone, naphtol, naphtylamine, aminothiazole, thiophène, hydroxypyridine ;
- X et Y représentent, indépendamment l'un de l'autre un atome d'azote ou un groupe CR' avec R' représentant un atome d'hydrogène ou un groupe alkyle comportant de 1 à 6 atomes de carbone;
- A est un contre-ion anionique.

2. Composition de teinture selon la revendication 1, dans laquelle le groupe R⁶ est un groupe cationique.

3. Composition de teinture selon la revendication 1 ou 2, dans laquelle le groupe R⁶ est un groupe N-alkyl(C1-C4)pyridinium, un groupe N-alkyl(C1-C4)imidazolium, un groupe N-alkyl(C1-C4)triazolium.

4. Composition de teinture selon l'une quelconque des revendications 1 à 3, dans laquelle ledit colorant est choisi parmi les composés de formules (II) et (III) suivantes : 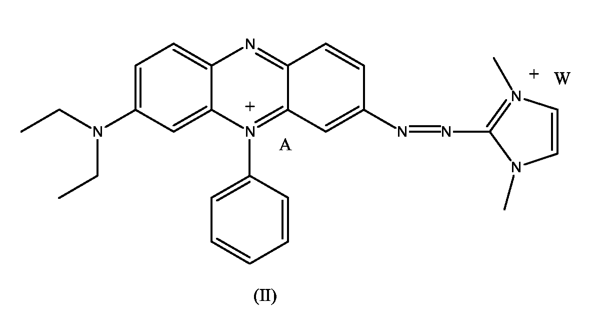 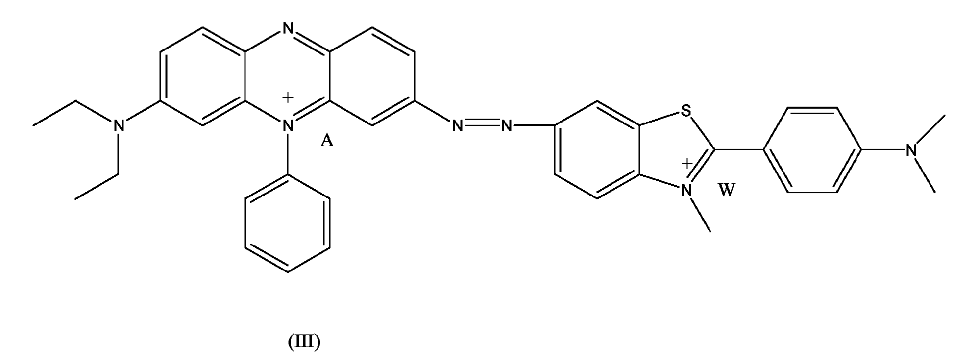 dans lesquelles A est tel que défini dans la revendication 1 et W représente un contre-ion anionique.

5. Composition de teinture selon la revendication 4, dans laquelle A et W sont des ions chlorure.

6. Composition de teinture selon l'une quelconque des revendications 1 à 5, dans laquelle ledit ou lesdits colorant(s) est (sont) présent(s) en une teneur allant de 0,001 à 20 % en poids du poids total de la composition.

7. Composition de teinture selon l'une quelconque des revendications 1 à 6, dans laquelle ledit ou lesdits colorant(s) est (sont) présent(s) en une teneur allant de 0,01 à 10 % en poids, et encore plus préférentiellement entre 0,1 et 5 % en poids du poids total de la composition.

8. Composition de teinture selon l'une quelconque des revendications 1 à 7, comprenant un milieu aqueux constitué par de l'eau ou par un mélange d'eau et d'un solvant organique acceptable sur le plan cosmétique.

9. Composition de teinture selon la revendication 8, dans laquelle ledit solvant organique acceptable sur le plan cosmétique est choisi parmi les alcools tels que l'alcool éthylique, l'alcool isopropylique, l'alcool benzylique, les polyols, les éthers de polyols, les alcanes, les cétones et les mélanges de ceux-ci.

10. Composition de teinture selon l'une quelconque des revendications 1 à 9, comprenant, en outre, un ou plusieurs colorants directs différents des composés de formule (I) tels que définis dans la revendication 1.

11. Composition de teinture selon la revendication 10, dans laquelle le ou les colorants directs différents des composés de formule (I) sont choisis parmi les colorants nitrés, les méthines, les azométhines, les styriles, les triarylméthanes, les diarylméthanes, les colorants azoïques, les colorants anthraquinoniques et naphtoquinoniques, les porphyrines, les tétraphénylporphyrines, les métalloporphyrines, les phtalocyanines, les colorants naturels de type caroténoïdes, terpénoïdes, flavonoïdes, les colorants fluorescents tels que la fluorescéine, la rhodamine, la coumarine.

12. Composition de teinture selon l'une quelconque des revendications 1 à 11, comprenant, en outre, une ou plusieurs bases d'oxydation associées éventuellement à un ou plusieurs coupleurs.

13. Composition de teinture selon la revendication 12, dans laquelle la ou les bases d'oxydation sont choisies parmi les paraphénylènediamines, les bisphénylalkylènediamines, les paraaminophénols, les orthoaminophénols, les bases hétérocycliques.

14. Composition de teinture selon la revendication 12, dans laquelle le ou les coupleurs sont choisis parmi les coupleurs métaphénylènediamines, les coupleurs métaaminophénols, les coupleurs métadiphénols, les coupleurs naphtaléniques, les coupleurs hétérocycliques.

15. Composition selon l'une quelconque des revendications 1 à 14, comprenant, en outre, un ou plusieurs additifs choisis parmi les agents tensioactifs, les agents épaississants, les agents anti-oxydants, les agents séquestrants, les agents dispersants, les agents de conditionnement du cheveu, les agents conservateurs, les agents opacifiants, les agents alcalinisants, les agents acidifiants, les parfums.

16. Composition de teinture selon l'une quelconque des revendications 1 à 15, présentant un pH compris entre 3 et 12 environ, de préférence entre 5 et 11 et encore plus préférentiellement entre 6 et 10.

17. Utilisation de composés de formule (I) tels que définis dans la revendication 1 en tant que colorant direct dans des compositions de teinture des fibres kératiniques.

18. Procédé de coloration directe de fibres kératiniques comprenant successivement les étapes suivantes :
a) appliquer sur les fibres kératiniques une composition de teinture telle que définie selon l'une quelconque des revendications 1 à 16;
b) laisser reposer la composition sur les fibres kératiniques pendant un temps suffisant pour obtenir la coloration;
c) rincer éventuellement les fibres kératiniques pour en éliminer ladite composition de teinture;
d) laver éventuellement les fibres kératiniques une ou plusieurs fois, les rincer après chaque lavage ;
e) les sécher.

19. Procédé de coloration des fibres kératiniques comprenant successivement les étapes suivantes :
f) appliquer sur les fibres kératiniques une composition de teinture comprenant au moins un composé de formule (I) telle que définie selon l'une quelconque des revendications 1 à 16 et au moins un colorant d'oxydation, la couleur du colorant d'oxydation étant révélée par un agent oxydant ;
g) laisser reposer la composition sur les fibres kératiniques pendant un temps suffisant pour obtenir la coloration;
h) rincer éventuellement les fibres kératiniques pour en éliminer ladite composition de teinture;
i) laver éventuellement les fibres kératiniques une ou plusieurs fois, les rincer après chaque lavage ;
j) les sécher.

20. Procédé de coloration selon la revendication 18 ou 19, dans lequel le temps de repos est de 3 à 60 minutes, de préférence de 5 à 40 minutes, et plus préférentiellement encore de 15 à 30 minutes.

21. Procédé de coloration selon la revendication 19, dans lequel l'agent oxydant est choisi parmi le peroxyde d'hydrogène, le peroxyde d'urée, les bromates de métaux alcalins, les persels tels que les perborates et persulfates, les peracides et les enzymes oxydases telles que les peroxydases, les oxydoréductases à deux électrons telles que les uricases et les oxygénases à quatre électrons telles que les laccases.
